# EUROPÄISCHE PATENTANMELDUNG

(11) **EP 1 243 288 A2**
(43) Veröffentlichungstag der Anmeldung: **25.09.2002**
(21) Anmeldenummer: 02004837.7
(22) Anmeldetag: 03.03.2002
(51) Int. Cl.: A61N 5/06

(54) **Kosmetische oder medizinische Bestrahlungseinrichtung**

(30) Priorität: 06.03.2001 DE 10110686
(71) Anmelder: Kopper, Iris, 44227 Dortmund (DE)
(72) Erfinder: Die Erfindernennung liegt noch nicht vor
(74) Vertreter: Grosse, Wolf-Dietrich, Dipl.-Ing.

(57) **Zusammenfassung**

Eine kosmetische oder medizinische Bestrahlungseinrichtung, die aus lichtdurchlässigen. Auflageflächen für die zu bestrahlenden Personen aufweisenden Tragplatten (TR) besteht, die mit Abstand oberhalb dieser Personen angeordnet sind und rückseitig von Strahlungslichtquellen (SQ) mit Reflektionsspiegeln (RS) beaufschlagt sind, deren Spiegelflächen (SPF) eine, den Tragplatten (TR) konkav zugewandte Wölbung aufweisen. Der konkave verlauf der Spiegelflächen (SPF) entspricht dabei etwa dem eines Abschnitts einer archimedischen oder logarithmischen Spirale, deren Pol in dem Mittelpunkt bzw. der Mittenachse der Strahlungslichtquelle (SQ) liegt.

## Beschreibung

Die Erfindung bezieht sich auf eine kosmetische oder medizinische Bestrahlungseinrichtung, bestehend aus, lichtdurchlässige Auflageflächen für die zu bestrahlenden Personen aufweisenden oder mit Abstand oberhalb dieser angeordneten Tragplatten und, diese Tragplatten rückseitig beaufschlagenden Strahlungslichtquellen mit Reflektionsspiegeln, deren Spiegelflächen eine, den Tragplatten konkav zugewandte Wölbung aufweisen.

Bestrahlungseinrichtungen dieser Art werden insb. bei Bestrahlungsliegen verwendet, die eine ebene oder, den Körperformen angepaßte Auflagefläche für die zu bestrahlende Person aufweisen. Diese Auflageflächen werden von Tragplatten aus lichtdurchlässigem Material gebildet, die rückseitig, von den Strahlen der Strahlungslichtquellen beaufschlagt werden. Die Bestrahlungsliegen weisen durchweg weitere, entsprechend ausgebildete Tragplatten und besondere Strahleinrichtungen auf, die mit Abstand über und ggf. seitlich der zu bestrahlenden Person angeordnet sind.

Als Strahlungslichtquellen werden bei diesen Einrichtungen durchweg Leuchtröhren mit hinter diesen oder in die Röhren integrierten Reflektionsspiegeln verwendet. Die Nachteile dieser Bauart bestehen einmal darin, daß ein Teil der rückseitigen Strahlung in den Röhren verloren geht und für die jeweils zu bestrahlende Fläche eine verhältnismäßig große Anzahl solcher, dabei dicht nebeneinander angeordneten Leuchtröhren benötigt wird, und dabei die, hier glatten Spiegelflächen, der Reflektionsspiegel wenig wirksam sind. Hinzu kommt der durch die große Anzahl der Röhren bedingte erhebliche Aufwand an elektrischen Installationselementen, wie Fassungen, Vorschaltgeräten, Startern und der Verdrahtung sowie die große Wärmeentwicklung, die entsprechende Kühleinrichtungen erfordert und der damit verbundene hohe Stromverbrauch.

Der Erfindung liegt die Aufgabe zugrunde, diesen Aufwand an Röhren, Spiegeln, Installationselementen und den Energieverbrauch erheblich zu verringern und dabei gleichzeitig eine gleichmäßigere Verteilung der Bestrahlung zu erzielen.

Diese Aufgabe wird dadurch gelöst, daß bei den verwendeten Reflektionsspiegeln der konkave Verlauf der Spiegelflächen etwa dem eines Abschnitts einer archimedischen oder logarithmischen Spirale entspricht, deren Pol in dem Mittelpunkt bzw. der Mittenachse der verwendeten Strahlungslichtquelle liegt. Bei Bestrahlungseinrichtungen, die aus einer Mehrzahl von parallel, mit Abständen nebeneinander in Reihen angeordneten Strahlungslichtquellen und diesen zugeordneten rinnenförmigen Reflektionsspiegeln bestehen, können, wie die Erfindung vorsieht, zwei Reflektionsspiegelrinnen, mit ihren Spiegelflächen einander zugewandt und mit ihren benachbarten Rinnenrändern aneinanderliegend, gemeinsam eine Reflektionsspiegelrinne mit einem symmetrischen, nach außen, den aneinander liegenden Rinnenrändern abgewandten, offenen Rinnenquerschnitt bilden, in dessen Symmetrie-Mittenlinie die Mitte der Strahlungslichtquelle liegt. Die Strahlungslichtquellen mit den zugeordneten Reflektionsspiegeln können dabei in Raumbereichen, seitlich der Tragplatten, unterhalb der Ebene von deren Auflageflächen bzw. oberhalb der darüber befindlichen Tragplatte angeordnet sein. Weiter können, wie die Erfindung vorsieht, die Reflektionsspiegel konkav gewölbt verlaufende Verlängerungen aufweisen, die in die Raumbereiche unterhalb der Auflageflächen bzw. oberhalb der darüber befindlichen Tragplatte hineinragen. Diese Verlängerungen können aus zwei zusammenhängend aufeinanderfolgenden Abschnitten bestehen, von denen der erste durch den Raumbereich, seitlich der Tragplatten und der zweite in den Bereich unter- bzw. oberhalb der Tragplatten geführt wird. Eine weitere Möglichkeit der Ausbildung der Bestrahlungseinrichtung besteht darin, daß bei Anordnung von Paaren einander gegenüberliegender Strahlungslichtquellen beiderseits einer Tragplatte, die Endkanten der Verlängerungen der Reflektionsspiegel, miteinander verbunden, eine auf die Tragplatte gerichtete, mit Abstand vor dieser endende Spitzkante bilden.

Die erfindungsgemäße Ausbildung und Anordnung der Strahlungslichtquellen und der Reflektionsspiegel erlaubt nicht nur die Verwendung von Hochleistungslichtquellen mit hohem Wirkungsgrad anstelle der üblichen Leuchtröhren; sie bringt auch eine gleichmäßigere und indirekte Strahlenverteilung mit sich, da bei der erfindungsgemäßen Ausbildung matte, stark streuende Reflektionsspiegel verwendet werden können. Gleichzeitig wird die Strahlwärmebelastung der zu bestrahlenden Personen wesentlich verringert.

Die Erfindung wird anhand der in der Zeichnung dargestellten Ausführungsbeispiele näher erläutert. In der Zeichnung zeigen
- Fig. 1: die Anordnung mehrerer, unterhalb einer Tragplatte liegender Strahlungslichtquellen mit ihren Reflektionsspiegeln in vertikalem Radialschnitt in schematischer Darstellung,
- Fig. 2: eine Strahlungslichtquelle mit ihrem verlängerten Reflektionsspiegel im vertikalen Radialschnitt, unterhalb einer Tragplatte in schematischer Darstellung,
- Fig.3: eine Paar-Anordnung von Strahlungslichtquellen entsprechend Fig. 2 mit den Verlängerungen unter einer Tragplatte im Radialschnitt, ebenfalls in schematischer Darstellung, und
- Fig. 4: die Seitenansicht der Anordnung mehrerer Tragplatten in Liegenform in schematischer Darstellung.

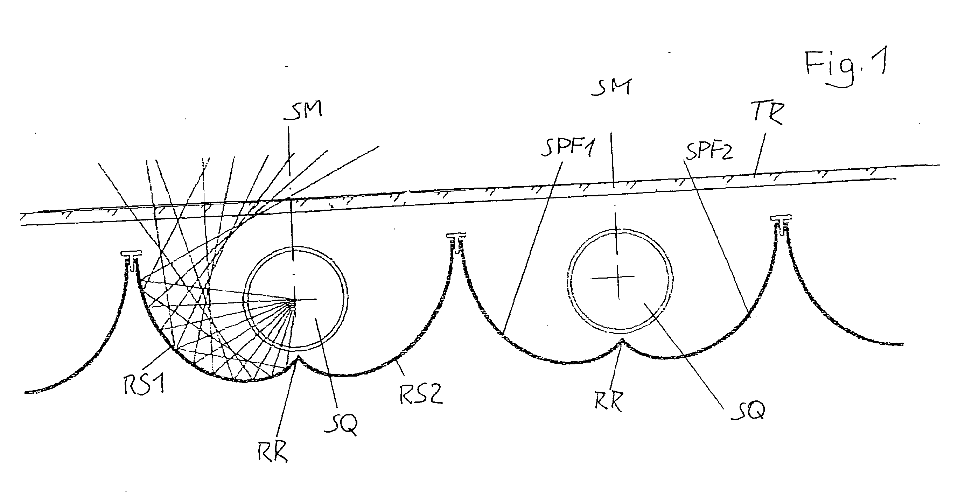

Wie aus Fig. 1 zu ersehen, sind unterhalb der Strahlungslichtquelle SQ jeweils zwei Reflektionsspiegel in der Form von Reflektionsspiegelrinnen RS1, RS2 angeordnet, deren Spiegelflächen SPF1 und SPF2, einander zugewandt und mit ihren benachbarten Rinnenrändern RR aneinanderliegend gemeinsam einen offenen Rinnenquerschnitt bilden. In der Symmetrie-Mittenlinie SM dieses zusammengesetzten Spiegels ist, mit Abstand über dem benachbarten Rinnenrand RR die Strahlungslichtquelle SQ angeordnet.

Diese Anordnung aus Reflektionsspiegeln und Strahlungslichtquelle befindet sich unter einer Tragplatte TR aus lichtdurchlässigem Werkstoff. Wie ersichtlich, wird bei dieser Anordnung fast die gesamte Strahlwirkung der Strahlungslichtquelle SQ direkt oder durch die beiden Reflektionsspiegel RS1, RS2 reflektiert, gegen die Tragplatte gerichtet und ausgenutzt.

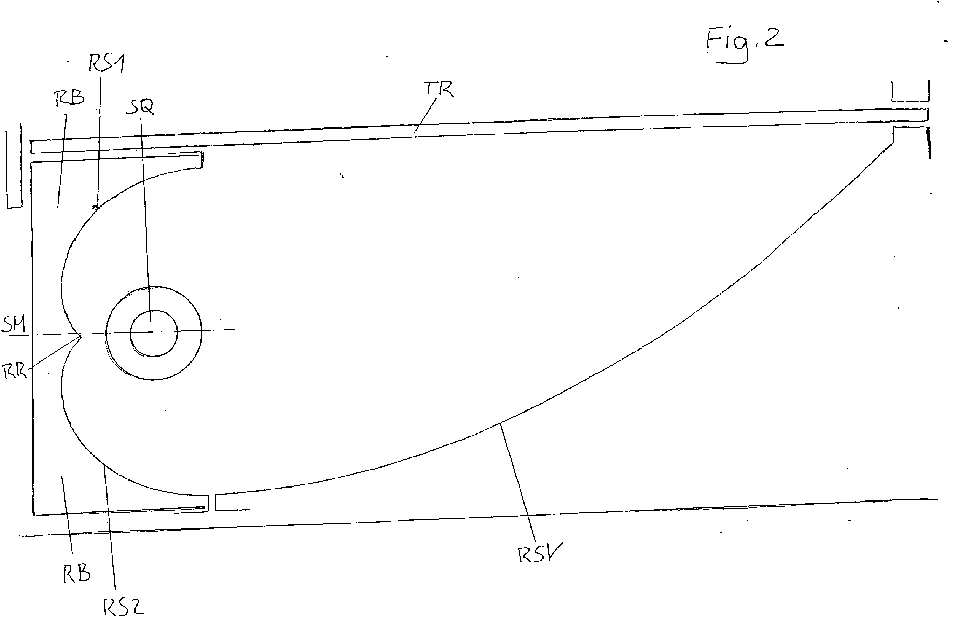

Bei der Ausbildung nach Fig. 2 ist die Spiegel-Strahlungslichtquellenanordnung nach Fig. 1 in einem Raumbereich RB seitlich der Tragplatte TR und unterhalb der Ebene von deren Auflagefläche für die, nicht dargestellte, zu bestrahlende Person angeordnet, wobei der untere der beiden Reflektionsspiegel RS2 eine der Tragplatte TR konkav zugewandte, gewölbt verlaufende Verlängerung RSV aufweist, die in den Raumbereich unterhalb der Tragplatte TR hineinragt. Diese Anordnung führt dazu, daß die auf der Auflagefläche liegende Person der von der Lichtquelle ausgehenden Strahlung nicht direkt, sondern nur indirekt, bei breitflächiger Verteilung der Strahlen ausgesetzt ist, womit auch partielle Wärmekonzentrationen vermieden werden.

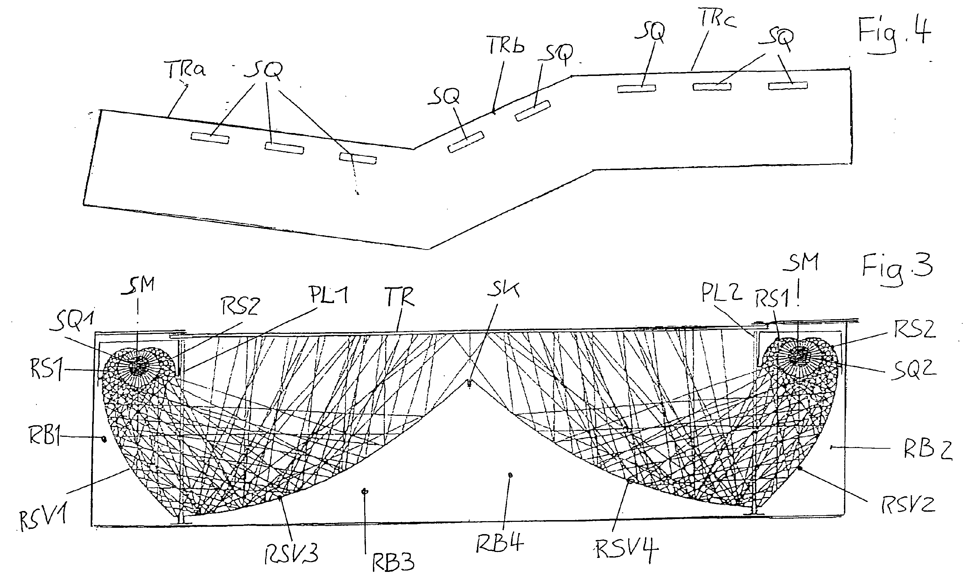

Aus Fig. 3 geht eine der Ausbildung nach Fig. 2 entsprechende Ausbildungsform hervor, bei der, in den mit Abstand einander gegenüberliegenden seitlichen Raumbereichen RB1 und RB2 die Strahlungslichtquellen SQ1; SQ2 mit ihren Reflektionsspiegeln RS1 und RS2 und deren erste Verlängerungen RSV1 bzw. RSV2 angeordnet sind. an diese schließen sich im Bereich einer vertikalen Platte PL1 bzw. PL2 aus lichtdurchlässigem Material jeweils ein zweiter Verlängerungsabschnitt RSV3 bzw. RSV4 an, der in dem Raum RB3; RB4 unterhalb der Tragplatte TR hineinragt. Die Endkanten dieser beiden Verlängerungen RSV3; RSV4 laufen in einer Spitzkante SK zusammen, die mit Abstand mittig unter der Tragplatte TR liegt.

Die beiden Anordnungen nach den Fig. 1, Fig. 2 und Fig. 3 können statt nach oben auch nach unten gerichtet, mit Abstand oberhalb einer Tragplatte angeordnet werden, um eine Bestrahlung der auf einer Tragplatte liegenden und von unten her bestrahlten Person auch von oben her zu bewirken.

Fig. 4 zeigt die seitliche Anordnung der Strahlungslichtquellen SQ bei einer aus mehreren Tragplatten TRa, TRb, TRc zusammengesetzten Tragplatte zu einer Liege mit geneigt zueinander verlaufenden Auflageflächen.

### Bezugszeichenliste

- TR: Tragplatte
- TRa: "
- TRb: "
- TRc: "
- SQ: Strahlungslichtquelle
- RS1: Reflektionsspiegelrinne
- RS2: "
- SPF1: Spiegelfläche
- SPF": "
- RR: Rinnenränder (benachbart)
- SM: Symetrie-Mittenlinie
- RB: Raumbereich
- RB1: "
- RB2: "
- RB3: "
- RB4: "
- RSV: Verlängerung (von RS)
- RSV1: "
- RSV2: "
- RSV3: "
- RSV4: "
- PL1: (vertikale) Platte
- PL2: "
- SK: Spitzkante

## Patentansprüche

1. Kosmetische oder medizinische Bestrahlungseinrichtung, bestehend aus, lichtdurchlässige Auflageflächen für die zu bestrahlenden Personen aufweisenden, mit Abstand oberhalb dieser angeordneten Tragplatten (TR) und diese Tragplatten (TR) rückseitig beaufschlagenden Strahlungslichtquellen (SQ) mit Reflektionsspiegeln (RS), deren Spiegelflächen (SPF) eine, den Tragplatten (TR) konkav zugewandte Wölbung aufweisen,
**dadurch gekennzeichnet,**
**daß** der konkave Verlauf der Spiegelflächen (SPF) etwa dem eines Abschnitts einer archimedischen oder logarithmischen Spirale entspricht, deren Pol in dem Mittelpunkt bzw. der Mittenachse der Strahlungslichtquelle (SQ) liegt.

2. Bestrahlungseinrichtung nach Anspruch 1,
bestehend aus einer Mehrzahl von parallel mit Abständen nebeneinander in Reihen angeordneter Strahlungslichtquellen (SQ) und, diesen zugeordneten rinnenförmigen Reflektionsspiegeln (RS),
**dadurch gekennzeichnet,**
**daß** zwei Reflektionsspiegelrinnen (RS1, RS2) mit ihren Spiegelflächen (SPF1, SPF2) einander zugewandt und mit ihren benachbarten Rinnenrändern (RR) aneinanderliegend, gemeinsam eine Reflektionsspiegelrinne mit einem symmetrischen, nach außen, den aneinander liegenden Rinnenrändern (RR) abgewandten, offenen Rinnenquerschnitt bilden, in dessen Symmetrie-Mittenlinie (SM) die Mitte der Strahlungslichtquelle (SQ) liegt.

3. Bestrahlungseinrichtung nach den Ansprüchen 1 und/oder 2,
**dadurch gekennzeichnet,**
**daß** die Strahlungslichtquelle (SQ) mit den zugeordneten Reflektionsspiegeln (RS1, RS2) in Raumbereichen (RB) seitlich der Tragplatten (TR), unterhalb der Ebene von deren Auflageflächen bzw. oberhalb der darüber befindlichen Tragplatte (TR) angeordnet sind.

4. Bestrahlungseinrichtung nach Anspruch 3,
**dadurch gekennzeichnet,**
**daß** die Reflektionsspiegel (RS2) konkav gewölbt verlaufende Verlängerungen (RSV) aufweisen, die in die Raumbereiche (RB) unterhalb der Auflageflächen bzw. oberhalb der darüber befindlichen Tragplatten (TR) hineinragen.

5. Bestrahlungseinrichtung nach den Ansprüchen 3 und/oder 4,
**dadurch gekennzeichnet,**
**daß** die Verlängerungen aus zwei zusammenhängend aufeinanderfolgenden Abschnitten (RSV1, RSV3 bzw. RSV2; RSV4) bestehen, von denen der erste durch die Raumbereiche (RB1; RB2) seitlich der Tragplatten (TR) und der zweite in die Raumbereiche (RB3, RB4) unter- bzw. oberhalb der Tragplatten (TR) geführt wird.

6. Bestrahlungseinrichtung nach den Ansprüchen 1 bis 5,
**dadurch gekennzeichnet,**
**daß** bei Anordnung von paarweise, in seitlich der Tragplatten (TR) einander gegenüberliegenden Raumbereichen (RB) angeordneten Strahlungslichtquellen, die Endkanten der Verlängerungen (RSV) der Reflektionsspiegel (RS), miteinander verbunden, eine auf die Tragplatte (TR) gerichtete, mit Abstand vor dieser endende Spitzkante (SK) bilden.
